(19) Europäisches Patentamt
European Patent Office
Office européen des brevets

(11) Numéro de publication: **0 160 582 A1**

(12) # DEMANDE DE BREVET EUROPEEN

(21) Numéro de dépôt: 85400352.2

(22) Date de dépôt: 26.02.85

(51) Int. Cl.⁴: **C 07 C 119/06**
A 61 K 31/22, A 61 K 31/195

(30) Priorité: 09.03.84 FR 8403686

(43) Date de publication de la demande: 06.11.85 Bulletin 85/45

(84) Etats contractants désignés: AT BE CH DE FR GB IT LI LU NL SE

(71) Demandeur: SYNTHELABO
58, rue de la Glacière
F-75621 Paris Cedex 13(FR)

(72) Inventeur: Raizon, Bernard
49, rue Gabriel Péri
F-91270 Vigneux(FR)

(72) Inventeur: Wick, Alexander
10, Boulevard des Plants
F-78860 St Nom La Breteche(FR)

(74) Mandataire: Thouret-Lemaitre, Elisabeth et al,
Service Brevets - SYNTHELABO 58, rue de la Glacière
F-75621 Paris Cedex 13(FR)

(54) **Dérivés d'acides w-(hydroxy-2 phényl) méthylèneamino-phényl-alcanecarboxyliques, leur préparation et leur application en thérapeutique.**

(57) Dérivés d'acides ω-[(hydroxy-2 phényl) méthylène-amino]-phényl-alcanecarboxyliques répondant à la formule

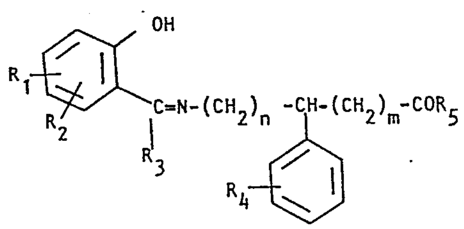

dans laquelle $R_1$ et $R_2$ représentent chacun l'hydrogène, un halogène ou un $(C_{1-4})$ alkyle, $R_3$ représente un $(C_{1-6})$ alkyle ou un phényle pouvant porter un halogène ou un $(C_{1-4})$ alkyle, $R_4$ représente un hydrogène ou un halogène ou un $(C_{1-4})$ alkyle, $R_5$ représente un hydroxy, O-métal alcalin ou alcalinoterreux, $(C_{1-4})$ alcoxy ou amino, n et m représentent 0, 1 ou 2.

Application en thérapeutique.

EP 0 160 582 A1

Croydon Printing Company Ltd

0160582

La présente invention concerne des dérivés d'acides ω-[(hydroxy-2 phényl) méthylène-amino]-phényl- alcanecarboxyliques, leur préparation et leur aplication en thérapeutique.

Les composés de l'invention répondent à la formule (I)

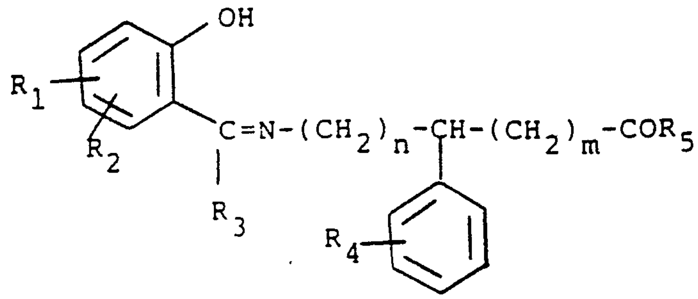

(I)

dans laquelle

$R_1$ et $R_2$ représentent chacun, indépendamment l'un de l'autre, un atome d'hydrogène, un atome d'halogène ou un radical $(C_{1-4})$alkyle,

$R_3$ représente un radical $(C_{1-6})$alkyle ou un radical phényle pouvant porter un atome d'halogène ou un radical $(C_{1-4})$alkyle,

$R_4$ représente un atome d'hydrogène ou d'halogène ou un radical $(C_{1-4})$alkyle,

$R_5$ représente un radical hydroxy, O-métal alcalin ou alcalinoterreux, $(C_{1-4})$alcoxy ou amino,

n et m représentent chacun un nombre entier égal à 0, 1 ou 2.

Les composés de l'invention possèdent un carbone asymétrique et peuvent exister sous la forme de racémates et d'isomères optiquement actifs qui font partie de l'invention.

Les composés de l'invention peuvent être préparés selon le schéma réactionnel suivant

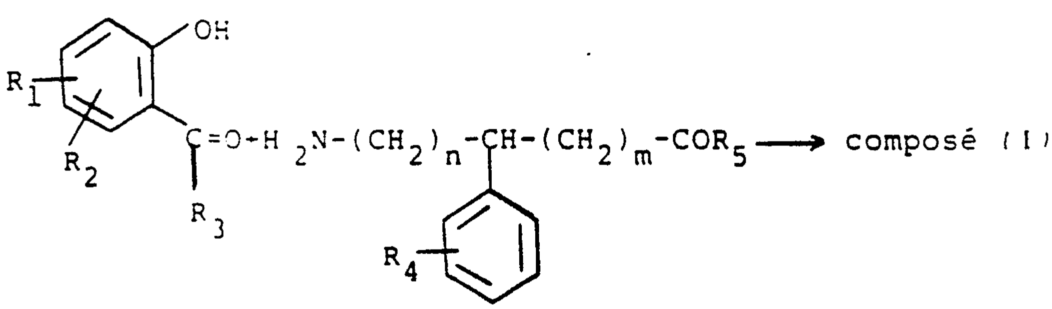


(II) (III)

Les radicaux des composés (II) et (III) ont les mêmes définitions que ci-dessus.

La réaction entre le composé (II) et le composé (III) est effectuée dans un solvant tel que le méthanol, à la température de reflux du solvant, en présence d'un alcoolate alcalin.

Les composés (II) sont soit décrits dans la littérature, soit décrits dans les précédents brevets de la demanderesse n° 75 24065, 81 21559 et 82 19981.

Les composés (III) sont décrits dans la littérature.

Il est bien sûr également possible de préparer à partir des acides (I) correspondants les amides (I) dans lesquels $R_5$ est $NH_2$, les esters (I) dans lesquels $R_5$ est un radical $(C_{1-4})$alcoxy et les sels (I) dans lesquels $R_5$ est un groupe O-alcalin ou O-alcalinoterreux, selon les méthodes classiques de la littérature.

Les exemples suivants illustrent la préparation des composés. Les analyses et les spectres IR et RMN confirment la structure des composés.

Exemple 1

Acide [[(chloro-5 hydroxy-2 phényl)méthylméthylène]amino]-4 (chloro-4 phényl)-3 butanoïque.

On évapore, à siccité, sous pression réduite, un mélange de 10 g (0,0586 mole) de chloro-4 hydroxy-2 acétophénone, 12,5 g (0,0585 mole) d'acide amino-4 (chloro-4 phényl)-3 butanoïque et de 3,2 g (0,0592 mole) de méthylate de sodium dans 600 ml de méthanol.

On évapore à siccité 3 fois 500 ml de méthanol. On refroidit, on dissout le résidu dans 1 litre d'eau, puis on acidifie à pH4 par de l'acide citrique.

Le produit précipite : on agite quelques instants, filtre sur fritté, essore et lave plusieurs fois le produit par de l'eau. On essore et sèche au dessiccateur en présence de $P_2O_5$.
On dissout le produit dans 1 litre de $CH_2$ $Cl_2$ et 400 ml de MeOH à chaud : on traite au charbon, filtre sur papier, concentre à 200 ml. Le produit précipite : on le filtre l'essore, et le lave plusieurs fois par de l'éther. On redissout à nouveau le produit dans 700 ml de $CH_2$ $Cl_2$ et 400 ml de MeOH (à chaud)

On concentre à environ 150 ml. Le produit précipite : on le filtre, l'essore, et le lave deux fois par 30 ml d'éther. On essore et sèche au dessiccateur à 80°C en présence de $P_2O_5$.

Le composé obtenu fond à 220-221°C.

Exemple 2

Acide [[(chloro-5 hydroxy-2 propyl-3 phényl)(méthyl-4 phényl)méthylène]amino]-4 (chloro-4 phényl)-3 butanoïque.

On évapore, à siccité, sous pression réduite, un mélange de 5 g (0,0173 mole) de (chloro-5 hydroxy-2 n-propyl-3 phényl) (méthyl-4 phényl) méthanone, de 3,69 g (0,0173 mole) d'acide amino-4 (chloro-4 phényl)-3 butanoïque, de 1 g (0,0185 mole) de méthylate de sodium et de 600 ml de méthanol.
On évapore 6 fois 500 ml de méthanol, on dissout le résidu dans 1,2 l d'eau et 0,200 l de $CH_2$ $Cl_2$, puis on acidifie à $pH_4$ avec de l'acide citrique. On extrait ensuite par trois fois 500 ml de $CH_2$ $Cl_2$, on lave la phase organique à l'eau, décante, sèche sur $MgSO_4$, filtre et évapore à sec.
Le produit précipite : on l'entraîne sur fritté par 100 ml d'éther de pétrole et on l'essore. On dissout le produit dans 300 ml d'éther et concentre à 100 ml. Le produit cristallise. On le filtre, l'essore et le sèche au dessiccateur. Le composé obtenu fond à 195 - 196°C.

Tableau

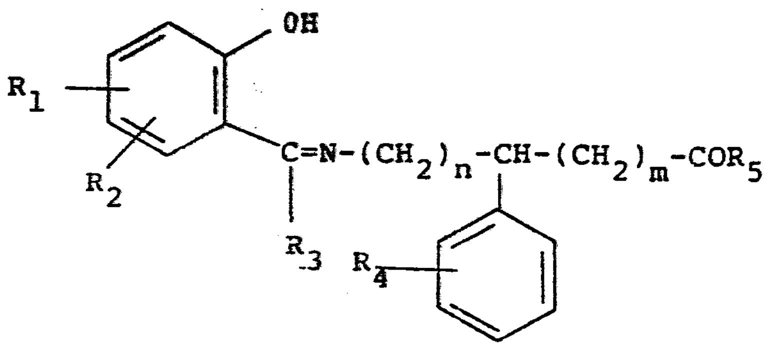

| Composé | $R_1$ | $R_2$ | $R_3$ | $R_4$ | $R_5$ | n | m | F(°C) |
|---|---|---|---|---|---|---|---|---|
| 1 | $3-CH_3$ | 5-Cl | $4-Cl-C_6H_4$ | H | OH | 0 | 2 | 181-2 |
| 2 | $3-CH_3$ | 5-Cl | $4-Cl-C_6H_4$ | H | $NH_2$ | 0 | 2 | 165-6 |
| 3 | $3-CH_3$ | 5-Cl | $4-Cl-C_6H_4$ | H | $NH_2$ | 1 | 1 | 167-8 |
| 4 | $3-CH_3$ | 5-Cl | $4-Cl-C_6H_4$ | H | OH | 1 | 1 | 81-2 |
| 5 | H | 5-Cl | $2-Cl-C_6H_4$ | H | $NH_2$ | 1 | 1 | 141-2 |
| 6 | $3-CH_3$ | 5-Cl | $4-Cl-C_6H_4$ | H | OH | 2 | 0 | 154-5 |
| 7 | H | 5-F | $4-Cl-C_6H_4$ | H | $NH_2$ | 1 | 1 | 165-6 |
| 8 | $3-CH_3$ | 5-Cl | $4-Cl-C_6H_4$ | H | $NH_2$ | 2 | 0 | 174-5 |
| 9 | $3-C_3H_7$ | 5-Cl | $4-Cl-C_6H_4$ | 4-Cl | OH | 1 | 1 | 156-7 |
| 10 | $3-CH_3$ | 5-Cl | $4-Cl-C_6H_4$ | 4-Cl | OH | 1 | 1 | 203-4 |
| 11 | $3-C_3H_7$ | 5-Cl | $4-CH_3-C_6H_4$ | 4-Cl | OH | 1 | 1 | 195-6 |
| 12 | H | 5-Cl | $CH_3$ | 4-Cl | OH | 1 | 1 | 220-1 |
| 13 | H | 5-Cl | $C_2H_5$ | 4-Cl | OH | 1 | 1 | 173-4 |

Tableau (suite)



| Composé | $R_1$ | $R_2$ | $R_3$ | $R_4$ | $R_5$ | n | m | F(°C) |
|---|---|---|---|---|---|---|---|---|
| 14 | H | 5-Cl | $CH_3$ | 4-Cl | $OCH_3$ | 1 | 1 | huile $n_D^{26}$ = 1,5940 |
| 15 | $3-CH_3$ | 5-Cl | $4-Cl-C_6H_4$ | 4-Cl | $OCH_3$ | 1 | 1 | huile $n_D^{26}$ = 1,6104 |
| 16 | $3-CH_3$ | 5-Cl | $C_6H_5$ | H | OH | 0 | 2 | 121-2 |
| 17 | $3-CH_3$ | 5-Cl | $CH_3$ | 4-Cl | OH | 1 | 1 | 203-4 |
| 18 | $3-CH_3$ | 5-Cl | $C_2H_5$ | 4-Cl | OH | 1 | 1 | 175-6 |
| 19 | $3-CH_3$ | 5-Cl | $CH_3$ | 4-Cl | $OCH_3$ | 1 | 1 | 75-6 |
| 20 | $3-C_2H_5$ | 5-Cl | $4-C_2H_5-C_6H_4$ | 4-Cl | OH | 1 | 1 | 170-170,5 |
| 21 | $3-nC_3H_7$ | 5-Cl | $C_2H_5$ | 4-Cl | OH | 1 | 1 | 178-9 |
| 22 | $3-CH_3$ | 5-Cl | $C_2H_5$ | 4-Cl | ONa | 1 | 1 | 135-6 |
| 23 | $3-_nC_3H_7$ | 5-Cl | $CH_3$ | 4-Cl | OH | 1 | 1 | 222-3 |
| 24 | H | 5-Cl | $nC_3H_7$ | 4-Cl | OH | 1 | 1 | 146-7 |
| 25 | $3-CH_3$ | 5-Cl | $C_2H_5$ | 4-Cl | $OC_2H_5$ | 1 | 1 | huile $n_D^{21,5}$ = 1,5770 |
| 26 | $3-CH_3$ | $5-CH_3$ | $4-Cl-C_6H_4$ | 4-Cl | OH | 1 | 1 | 185-6 |
| 27 | H | 5-Cl | $nC_5H_{11}$ | 4-Cl | OH | 1 | 1 | 116-7 |

Tableau (suite)

| Composé | $R_1$ | $R_2$ | $R_3$ | $R_4$ | $R_5$ | n | m | F(°C) |
|---|---|---|---|---|---|---|---|---|
| 28 | $3-C_2H_5$ | 5-Cl | $CH_3$ | 4-Cl | OH | 1 | 1 | 189-190 |
| 29 | $3-C_2H_5$ | 5-Cl | $C_2H_5$ | 4-Cl | OH | 1 | 1 | 160-1 |
| 30 | $3-C_2H_5$ | 5-Cl | $C_2H_5$ | 4-Cl | $OCH_3$ | 1 | 1 | 100-2 |
| 31 | $3-nC_3H_7$ | 5-Cl | $C_2H_5$ | 4-Cl | $O_nC_4H_9$ | 1 | 1 | huile $n_D^{23}$ = 1,5645 |
| 32 | $3-nC_3H_7$ | 5-Cl | $CH_3$ | 4-Cl | $O_nC_3H_7$ | 1 | 1 | huile $n_D^{23}$ = 1,5690 |
| 33 | $3-CH_3$ | 5-Cl | $4-CH_3-C_6H_4$ | 4-Cl | OH | 1 | 1 | 187-8 |
| 34 | H | $5-CH_3$ | $CH_3$ | 4-Cl | OH | 1 | 1 | 171-2 |
| 35 | H | $5-CH_3$ | $CH_3$ | 4-Cl | $OCH_3$ | 1 | 1 | 95-6 |
| 36 | $3-CH_3$ | 5-Cl | $4-CH_3-C_6H_4$ | 4-Cl | $OCH_3$ | 1 | 1 | 33-4 |
| 37 | $3-C_2H_5$ | 5-Cl | $4-C_2H_5-C_6H_4$ | 4-Cl | $OCH_3$ | 1 | 1 | 91-2 |

Les composés de l'invention ont été soumis à des essais pharmacologiques montrant leur activité sur le système nerveux central.

La toxicité aiguë des composés a été déterminée sur la souris, par voie orale ; la dose léthale 50 est supérieure à 1000 mg/kg.

L'activité des composés sur le réflexe de flexion a été étudiée sur des rats mâles Sprague-Dawley spinalisés (Charles River, France) pesant de 200 à 240 g.
24 heures avant le test, les rats sont anesthésiés avec de l'hydrate de chloral (400 mg/kg i.p.) et leur moelle épinière est sectionnée au niveau de la 6e - 8e vertèbre thoracique.
Pour le test, les rats spinalisés sont fixés sur une tablette, la patte gauche reposant sur un support. Le tendon d'Achille de cette patte est relié par un fil (sous une tension de 5 g) à une jauge de contrainte qui est elle-même reliée à un enregistreur.
Une électrode en acier inoxydable est introduite en sous cutané dans la voute plantaire. Le reflexe est provoqué par des trains de stimulations électriques d'intensité 7 mA - de durée 200 ms, avec une fréquence de 50 Hz toutes les 5 secondes, pendant 1 minute par un stimulateur électrique GRASS S-48.

Les produits à étudier sont injectés par voie intraveineuse sous la forme d'une suspension préparée dans du sérum physiologique additionné de Tween 80 à 0,1 %.

La DE 50, dose qui diminue la force du réflexe de 50 % par rapport à la période contrôle, varie de 1 à 10 mg/kg, i.v.

L'activité myorelaxante et sédative a également été évaluée, dans le test du fil de traction. Dans ce test un rat ou une souris témoin, suspendu(e) à un fil par les pattes antérieures, ramène au moins l'une des pattes postérieures sur le fil en moins de 10 s.

Si le traitement par un composé affecte le pouvoir de l'animal à ramener les pattes postérieures sur le fil en moins de 10 s, l'effet est considéré comme myorelaxant - sédatif.

A des groupes de 6 rats on injecte par voie intraveineuse des doses variables des composés de l'invention, en suspension dans du Tween 80 (à 0,1 %) et on note leur activité myorelaxante toutes les 10 minutes pendant 2 h. Le pourcentage d'animaux qui ne peuvent pas grimper est calculé pour chaque dose et chaque période de temps.

Pour les composés de l'invention, la DE 50 (dose qui empêche 50 % des animaux de grimper sur le fil) est supérieure à 10· ce qui montre que les composés de l'invention n'ont pas d'effet sédatif - myorelaxant.

Les composés de l'invention peuvent être utilisés pour le traitement de la spasticité et des problèmes de motricité.

Les composés de l'invention peuvent être présentés sous toute forme appropriée pour l'administration par voie orale ou parentérale. La posologie quotidienne peut aller de 10 à 200 mg.

Revendications pour les états contractants : BE, CH, DE, FR, GB, IT, LI, LU, NL, SE.

1. Dérivés d'acides ω -[(hydroxy-2 phényl) méthylène-amino] -phényl-alcanecarboxyliques, répondant à la formule (I)

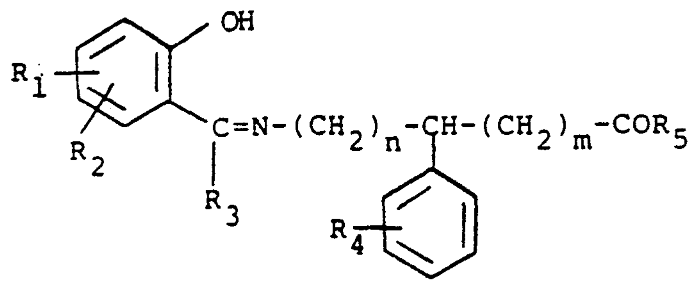

(I)

dans laquelle

$R_1$ et $R_2$ représentent chacun, indépendamment l'un de l'autre, un atome d'hydrogène, un atome d'halogène ou un radical $(C_{1-4})$alkyle,

$R_3$ représente un radical $(C_{1-6})$alkyle ou un radical phényle pouvant porter un atome d'halogène ou un radical $(C_{1-4})$alkyle,

$R_4$ représente un atome d'hydrogène ou d'halogène ou un radical $(C_{1-4})$alkyle,

$R_5$ représente un radical hydroxy, O-métal alcalin ou alcalinoterreux, $(C_{1-4})$alcoxy ou amino,

n et m représentent chacun un nombre entier égal à 0, 1 ou 2.

2. Composés selon la revendication 1, caractérisés par le fait que $R_1$ est en position 3.

3. Composés selon la revendication 2 caractérisés par le fait que $R_2$ est en position 5;

4. Composés selon la revendication 3, caractérisés par le fait que

$R_1$ est H ou un radical $(C_{1-4})$alkyle,

$R_2$ est un atome de fluor ou de chlore ou le radical méthyle,

$R_3$ est soit un radical phényle pouvant porter en position 4 un atome de chlore, le radical méthyle ou éthyle, soit un

radical méthyle, éthyle ou propyle,

$R_4$ est un atome d'hydrogène ou un atome de chlore en position 4,

$R_5$ est OH, ONa, $NH_2$ $OCH_3$ ou $OC_2H_5$,

n est égal à 0,1 ou 2

et

m est égal à 0, 1 ou 2.

5. L'acide [[(chloro-5 hydroxy-2 phényl)méthylméthylène] amino]-4 (chloro-4 phényl)-3 butanoïque.

6. L'acide [[(chloro-5 hydroxy-2 phényl) éthylméthylène] amino]-4 (chloro-4 phényl)-3 butanoïque.

7. Le [[(chloro-5 hydroxy-2 phényl)méthyl méthylène] amino]-4 (chloro-4 phényl)-3 butanoate de méthyle.

8. L'acide [[(chloro-5 hydroxy-2 méthyl-3 phényl) méthylméthylène] amino] -4(chloro-4 phényl)-3 butanoïque.

9. L'acide [[(chloro-5 hydroxy-2 n-propyl-3 phényl) (chloro-4 phényl)méthylène] amino]-4 (chloro-4 phényl)-3 butanoïque.

10. L'acide [[chloro-5 hydroxy-2 méthyl-3 phényl) (chloro-4 phényl)méthylène] amino] -4 (chloro-4 phényl)-3 butanoïque.

11. L'acide [[(chloro-5 hydroxy-2 n-propyl-3 phényl) (méthyl-4 phényl) méthylène] amino] -4 (chloro-4 phényl)-3 butanoïque.

12. Procédé de préparation des composés spécifiés dans la revendication 1, procédé caractérisé en ce que l'on fait réagir un composé de formule (II)

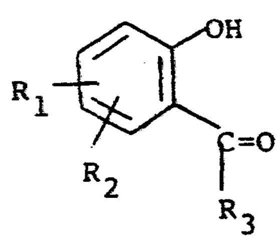

(II)

avec un composé de formule (III)

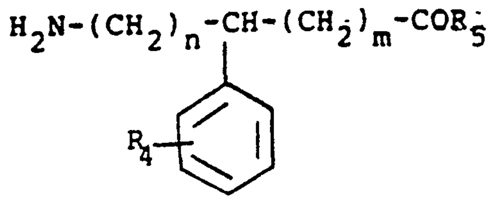

(III)

les radicaux ayant les significations données dans la revendication 1.

13. Médicament caractérisé en ce qu'il contient un composé tel que spécifié dans l'une quelconque des revendications 1 à 11

14. Composition pharmaceutique caractérisée en ce qu'elle contient un composé tel que spécifié dans l'une quelconque des revendications 1 à 8 en association avec tout excipient approprié.

<u>Revendication pour l'état contractant : AT.</u>

Procédé de préparation de dérivés d'acides ω-[(hydroxy-2 phényl) méthylène-amino]-phényl-alcanecarboxyliques, répondant à la formule (I)

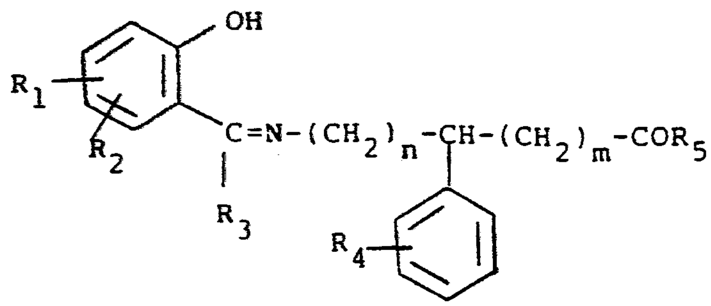

(I)

dans laquelle

$R_1$ et $R_2$ représentent chacun, indépendamment l'un de l'autre, un atome d'hydrogène, un atome d'halogène ou un radical $(C_{1-4})$alkyle,

$R_3$ représente un radical $(C_{1-6})$alkyle ou un radical phényle pouvant porter un atome d'halogène ou un radical $(C_{1-4})$alkyle,

$R_4$ représente un atome d'hydrogène ou d'halogène ou un radical $(C_{1-4})$alkyle,

$R_5$ représente un radical hydroxy, O-métal alcalin ou alcalinoterreux, $(C_{1-4})$alcoxy ou amino,

n et m représentent chacun un nombre entier égal à 0, 1 ou 2,procédé caractérisé en ce que l'on fait réagir un composé de formule (II)

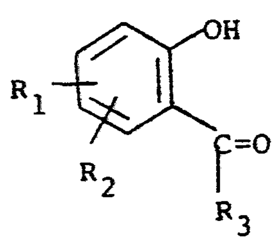

(II)

avec un composé de formule (III)

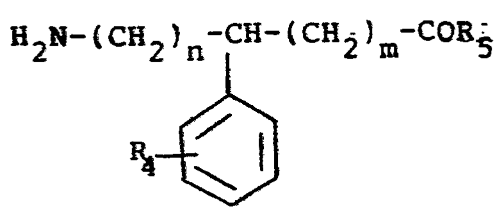

(III)

Office européen des brevets

**RAPPORT DE RECHERCHE EUROPEENNE**

0160582

Numéro de la demande

EP 85 40 0352

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | JOURNAL OF THE CHEMICAL SOCIETY, section C, 1968, pages 406-410, Londres, GB; A.F. AL-SAYYAB et al.: "Schiff Bases. Part I. Thermal decarboxylation of alpha-amino-acids in the presence of ketones" * Tableaux 1,2 * ----- | 1-3 | C 07 C 119/06<br>A 61 K 31/22<br>A 61 K 31/195 |
| | | | DOMAINES TECHNIQUES RECHERCHES (Int. Cl.4) |
| | | | C 07 C 119/00 |

Le présent rapport de recherche a été établi pour toutes les revendications

| Lieu de la recherche | Date d'achèvement de la recherche | Examinateur |
|---|---|---|
| LA HAYE | 21-06-1985 | GAUTIER R.H.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82